# EUROPEAN PATENT SPECIFICATION

(11) **EP 2 249 732 B1**
(45) Date of publication and mention of the grant of the patent: **14.09.2011**
(21) Application number: 08805037.2
(22) Date of filing: 03.10.2008
(51) Int. Cl.: A61B 18/00

(54) **PLASMA DEVICE FOR TREATING LIVING TISSUES**
PLASMAVORRICHTUNG ZUR BEHANDLUNG VON LEBENDEM GEWEBE
DISPOSITIF A PLASMA POUR TRAITER DES TISSUS VIVANTS

(30) Priority: 05.10.2007 IT RM20070521
(43) Date of publication of application: 17.11.2010
(73) Proprietor: Universita' Degli Studi Di Padova, 35122 Padova (IT); Consorzio RFX, 35127 Padova (IT)
(72) Inventor: LEONARDI, Andrea, I-35127 Padova (IT); DELIGIANNI, Velika, I-35122 Padova (IT); MARTINES, Emilio, I-35121 Padova (IT); ZUIN, Matteo, I-35122 Padova (IT); CAVAZZANA, Roberto, I-35125 Padova (IT); SERIANNI, Gianluigi, I-35020 Masera' Di Padova (IT); SPOLAORE, Monica, I-30035 Mirano (IT)
(74) Representative: Bardini, Marco Luigi
(86) International application number: PCT/EP2008/063275
(87) International publication number: WO 2009/043925

(56) References cited:
- WO-A-01/62169

## Description

### Field of the invention

The present invention relates to a device for treating living tissues based on plasma technology.

### State of the art

The use of plasma is already known for treating living tissues. Specifically, one use is to achieve a loss in mutual adhesion between cells and in the attachment thereof to their substrate by effect of the interaction between plasma and cells. Another use consists in inducing programmed cell death (apoptosis).

Finally, a further use is to sterilise the treated area by killing bacteria therein without significantly damaging the cells forming the area.

Either one or the other effect is obtained by varying the current, the voltage and the frequency in the plasma devices as well as the duration of the treatment.

Specifically, the plasma devices provided by the known art are based on the outflow of an inert gas that flows through a nozzle or hollow needle, so that an electric discharge of the "glow discharge" type or of the "electric arc" type is generated between said needle and the surface to be treated, as these are set at different potentials. The build-up of the electric discharge is allowed by the ionization of a fraction of the gas, that is by the generation of plasma. In turn, it is the current flow that maintains the process of plasma generation over time through the ionization of the neutral atoms by the electrons carrying such a current.

Said devices are defined having a unipolar configuration, as said needle represents an electrode, whereas the surface to be treated forms the other electrode of the circuit that is therefore established, whereby the plasma is generated outside the device and the treatment takes place by direct contact, or nearly, of the plasma with the tissue to be treated.

It is therefore apparent that the potential of the second electrode is not irrelevant. Specifically, the circuit consisting of the needle/tissue does not have a reference potential, with the subsequent risk of a possible generation of electric discharges concentrated between the needle and the tissue with the subsequent damaging of the tissue itself.

Another problem depends on the fact that, as the production of plasma is associated to a gas heating, when it is produced externally, the gas flow results potentially detrimental for the substrate to be treated.

A known device according to the preamble of claim 1 is disclosed in PCT international publication n. WO 01/62169. This known apparatus for skin resurfacing or cancerous tissue removal operates thanks to a release of thermal energy by applying voltage pulses in the range of hundreds of Watts. Consequently, the tissue suffers a thermal stress during the treatment.

### Summary of the invention

It is the object of the present invention to overcome all of the aforesaid drawbacks and to provide a device for treating living tissues for the purpose of obtaining effects such as sterilization, cell detachment, cell apoptosis or tissue necrosis, as well as other possible non-destructive interactions.

The object of the present invention is a plasma device for treating living tissues according to claim 1.

Said device may be applied in the sterilization of tissues, avoiding the damaging of the possible substrate, as the aforesaid device forms a bipolar system, including at least one pair of electrodes required for the generation of plasma. Specifically, the two electrodes are connected to an electric radiofrequency generator, and one of the two electrodes is connected to a reference potential that may be equivalent to ground.

Accordingly, the plasma is produced within the device itself, from which a flow exits substantially consisting of neutral atoms or molecules and active chemical species, thereby avoiding that the electrodes come into contact with the substrate to be treated.

Advantageously, said device results safe due to the absence of electric discharges between the device and the tissue, which therefore does not represent the second electrode and, furthermore, a low temperature of the gas is ensured, it being ideal for the substrate not to be damaged during treatment.

Therefore, a specific object of the present invention is a plasma device for treating living tissues, as described in greater detail in the claims. The dependent claims disclose preferred embodiments of the invention.

### Brief description of the Drawings

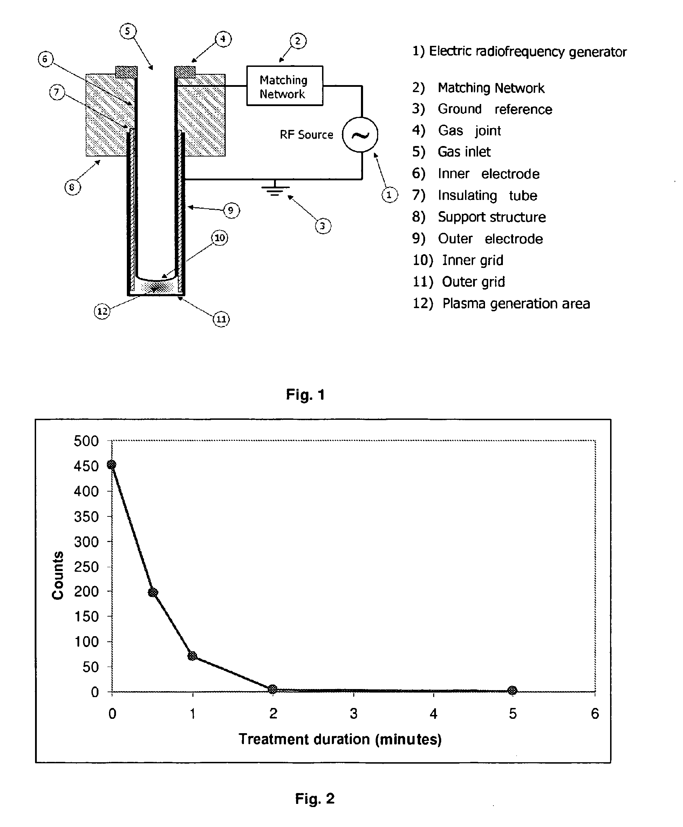

Further features and advantages of the invention will become more apparent in light of the detailed description of a preferred though not exclusive embodiment of a plasma device for treating living tissues, which is shown by way of non-limitative example with the aid of the accompanying drawings in which: Figure 1 shows the schematic diagram of a plasma device; Figure 2 shows the decreasing trend of the number of bacterial colonies as the treatment time increases with the present device, with a 90% reduction in a treatment of about 1 minute.

### Detailed description of a preferred embodiment of the invention

A preferred embodiment of the device, which is shown in Figure 1, provides for a pair of electrodes 6 and 9 consisting of two concentric nozzles, one inserted into the other and reciprocally insulated by means of an tube 7 made of insulating material, which is placed between the two and is as long as the electrode 9. The inner nozzle 6 forms the duct through which the gas flows and ends internally to the outer nozzle 9, so that a special area 12 is formed for the generation of plasma. Therefore, the latter results between said tube 7 made of insulating material and the inlets of both electrodes.

The inlet of the nozzle forming the inner electrode 6 is overlapped by a grid 10 made of conductive material and the inlet of the nozzle forming the outer electrode 9 is overlapped by a grid 11 made of conductive material. Both grids are galvanically connected with respective electrodes. Said grids are adapted to improve the generation of plasma and with reference to the specific application, they are from a few tenths of millimetre to some tens of millimetre away from one another.

Said electrodes and therefore said grids are subjected to a potential difference, as they are electrically connected to said electric generator 1, which is specifically an electric radiofrequency generator, by means of a coupling circuit 2, designated as Matching Network, which serves to minimise the power reflected towards the electric generator and to increase the voltage to the required values to ionize the gas.

Among the two electrodes, it is preferred that the electrode 9 and the grid 11 are galvanically connected to ground potential.

The free end of the inner electrode 6 is provided with a bayonet joint 5 of the known type, for an easy connection of the device to said gas source.

Both grids may be flat, concave or convex, although it is preferred for the grid 11 to be flat and for the grid 10 to be convex, the convexity being towards the grid 11. The electrodes 6 and 9, which are insulated by the insulating tube 7, are held in position by a support structure 8.

Therefore, the gas flow 5 is introduced through said joint, which flows within the inner electrode 6 up to the end fitted with the grid 10. By flowing through the area 12, the gas is transformed into plasma by the effect of the electric field induced by the generator on the grids, which varies over time at a frequency between 1 and 30 MHz and displays a potential difference of the order of one kV.

The generator provides a power of the order of ten W, from 1 to 30 W, with an optimal value of 10 W, a considerable fraction of which is dissipated within the coupling circuit. The plasma flowing out from the device hits the cell substrate to be treated.

Some preferred gases are helium, argon mixed with air, oxygen, nitrogen and mixtures thereof and/or other gases, to form a flow in the range between 0.1 and 5 litres/minute. Specifically, the use of noble gases may be avoided, for example, by increasing the potential difference between the electrodes.

Therefore, said device may be applied, among other uses, to treatments aimed at the sterilization of the substrate, for example, for the therapy or the prevention of infections, to treatments aimed at detaching cells from the substrate, and to treatments to induce cell apoptosis.

A preferred application method provides that the optimum distance between the outer grid 11 and the substrate to be treated varies between 0 and 2 cm, whereas the optimum application time is in the range between 10 seconds and 5 minutes. Advantageously, the low value of the power coupled with the plasma ensures that the latter is formed without the gas flow significantly heating. Thereby, the plasma results poorly ionized, i.e. most of the gas remains in the form of neutral atoms or molecules and in a non-equilibrium state, characterised by an electronic population at high temperature, that is of the order of some electronvolts, thus of some tens of thousands of Kelvins, and a ion population at room temperature. A plasma displaying these features is usually defined as a "low temperature plasma". Such a condition allows to prevent the occurrence of any thermal damage on the substrate to be treated.

Furthermore, including at least one pair of electrodes subjected to a known potential difference - one electrode (preferably the outer one) being set at a ground potential - ensures the total absence of electric discharges between the device and the substrate, thus avoiding the subsequent damaging thereof.

### Experimental results with an application in the field of ophthalmology.

The device has been applied for times varying between 30 seconds and 5 minutes to bacterial suspensions in Luria placed in wells. Some untreated control samples have been kept in the same temperature conditions as those exposed to plasma. After the treatment, each sample has been plated on an appropriate agar substrate and incubated at 37°C for sixteen hours. The number of colonies grown on each culture plate has been considered representative of the number of bacteria that survived the treatment. The tests have been carried out on Escherichia Coli. The graph represents the number of residual bacterial colonies as the exposure time varies (the value for t = 0 is that relative to the control samples). In Figure 2, a clear decrease may be noted in the number of colonies as the treatment time increases with a reduction of 90% in about 1 minute.

A treatment has also been carried out on a pig's eye on which a bacterial corneal ulcer had previously been induced. In this test, a 70% reduction in the bacterial charge has been noted as compared to control samples, after a treatment of 2 minutes, whereas the histological test under an optical microscope has not highlighted alterations of the cell structure of the corneal surface.

The specific modes to embody the invention described herein do not limit the content of this application, which includes all of the variants of the invention according to the claims.

## Claims

1. A plasma device for treating living tissues including:
- a pair of electrodes (6 and 9), one of which is connected to a reference potential;
- an electric generator (1) connected to said electrodes, adapted to generate a potential difference varying over time therebetween;
- a duct, one end of which is connected to a gas source and the other end of which contains said electrodes, so that the plasma is generated just before exiting the duct, **characterized in that** said electric generator is a radiofrequency electric generator having a power in the range between 1 and 30 W.

2. A device according to claim 1, wherein said gas source provides a gas mixture.

3. A device according to claim 2, wherein said gas mixture includes noble gases, such as argon and/or helium and/or neon.

4. A device according to claim 2, wherein said gas mixture includes reactive gases such as oxygen and/or nitrogen and/or steam and/or other gases according to the specific applications.

5. A device according to claim 1, wherein said pair of electrodes are an inner and an outer electrode, which are electrically insulated one from the other by means of an insulating tube (7) and are held in position by a support structure (8), whereby said pair define two concentric nozzles (6,9).

6. A device according to claim 1, wherein said electric generator (1) is a high frequency generator.

7. A device according to claim 1, further provided with a coupling circuit (2) that serves to minimize the power reflected towards the electric generator (1) and to increase the voltage up to values adapted to ionize the gas.

8. A device according to claim 1, wherein said reference potential is ground.

9. A device according to claim 5, wherein said inner electrode (6) has an inlet which results internal to the outer electrode (9).

10. A device according to claim 5, wherein the inlet of the inner electrode (6) is overlapped by a grid made of conductive material (10) galvanically connected to the inner electrode

11. A device according to claim 5, wherein said outer electrode (9) has an inlet which is overlapped by a grid made of conductive material (11) galvanically connected to the outer electrode.

12. A device according to claim 10 or 11, wherein said grids (10, 11) are flat or concave or convex.

13. A device according to claim 12, wherein the distance between said grids is in the range between a few tenths of millimetre and some tens of millimetre.

14. A device according to claim 12, wherein said outer grid (11) is flat and said inner grid (10) is convex, the convexity being towards the outer grid (11)

15. A device according to claim 5, wherein said insulating tube (7) is as long as the nozzle forming the outer electrode (9).

16. A device according to claim 1, wherein said electric generator has an optimum power of 10 W.

17. A device according to claim 7, wherein the set comprised of the electric generator and said coupling circuit is adapted to generate a potential difference between said electrode of the order of kV and a frequency between 1 and 30 MHz.

18. A device according to claim 1, wherein the gas flow flowing through said duct is in the range between 0,1 and 5 litres/minute.

## Patentansprüche

1. Plasma-Vorrichtung zur Behandlung von lebendem Gewebe, enthaltend:
- ein Paar Elektroden (6 und 9), von denen eine mit einem Referenzpotential verbunden ist;
- einen mit den Elektroden verbundenen elektrischen Generator (1), der ausgelegt ist, um eine Potentialdifferenz zu generieren, die über Zeit dazwischen variiert;
- eine Leitung, von der ein Ende mit einer Gasquelle verbunden ist und von der das andere Ende die Elektroden enthält, so dass das Plasma direkt vor dem Verlassen der Leitung erzeugt wird,
**dadurch gekennzeichnet, dass** der elektrische Generator ein elektrischer Radiofrequez-Generator ist, der eine Leistung im Bereich zwischen 1 und 30 W hat.

2. Vorrichtung nach Anspruch 1, wobei die Gasquelle eine Gasmixtur bereitstellt.

3. Vorrichtung nach Anspruch 2, wobei die Gasmixtur Edelgase, wie Argon und/oder Helium und/oder Neon, enthält.

4. Vorrichtung nach Anspruch 2, wobei die Gasmixtur reaktive Gase, wie Sauerstoff und/oder Stickstoff und/oder Dampf und/oder andere Gase entsprechend den spezifischen Anwendungen, enthält.

5. Vorrichtung nach Anspruch 1, wobei es sich bei dem genannten Paar Elektroden um eine innere und eine äußere Elektrode handelt, die eine von der anderen durch einen isolierenden Schlauch (7) elektrisch isoliert sind und von einer Stützstruktur (8) in Position gehalten werden, wobei das Paar zwei konzentrische Düsen definiert.

6. Vorrichtung nach Anspruch 1, wobei es sich bei dem elektrischen Generator (1) um einen Hochfrequenz-Generator handelt.

7. Vorrichtung nach Anspruch 1, ferner versehen mit einer Kopplungsschaltung (2), die dazu dient, um die zum elektrischen Generator (1) reflektierte Leistung zu minimieren und die Spannung auf Werte zu erhöhen, die dazu ausgelegt sind, das Gas zu ionisieren.

8. Vorrichtung nach Anspruch 1, wobei das genannte Referenzpotential Erde ist.

9. Vorrichtung nach Anspruch 5, wobei die innere Elektrode (6) einen Einlass hat, der im inneren der äußeren Elektrode (9) endet.

10. Vorrichtung nach Anspruch 5, wobei der Einlass der inneren Elektrode (6) von einem Gitter überlappt wird, das aus leitendem Material (10) besteht, das mit der inneren Elektrode galvanisch verbunden ist.

11. Vorrichtung nach Anspruch 5, wobei die äußere Elektrode (9) einen Einlass hat, der von einem Gitter überlappt wird, das aus leitendem Material (11) besteht, das mit der äußeren Elektrode galvanisch verbunden ist.

12. Vorrichtung nach Anspruch 10 oder 11, wobei die Gitter (10, 11) flach oder konkav oder konvex sind.

13. Vorrichtung nach Anspruch 12, wobei der Abstand zwischen den Gittern im Bereich zwischen einigen Zehntel Millimetern und einigen Zehn Millimetern liegt.

14. Vorrichtung nach Anspruch 12, wobei das äußere Gitter (11) flach ist und das innere Gitter (10) konvex ist, wobei die Wölbung zu dem äußeren Gitter (11) hin gerichtet ist.

15. Vorrichtung nach Anspruch 5, wobei der isolierende Schlauch (7) so lang wie die Düse ist, die die äußere Elektrode (9) formt.

16. Vorrichtung nach Anspruch 1 wobei der elektrische Generator eine optimale Leistung von 10 W hat.

17. Vorrichtung nach Anspruch 7, wobei das Set bestehend aus dem elektrischen Generator und der Kopplungsschaltung dazu ausgelegt ist, eine Potenzialdifferenz zwischen den Elektroden zu generieren, die im Bereich von kV liegt und eine Frequenz zwischen 1 und 30 MHz hat.

18. Vorrichtung nach Anspruch 1, wobei der Gasstrom, der durch die Leitung strömt, im Bereich zwischen 0,1 und 5 Liter/Minute liegt.

## Revendications

1. Un dispositif à plasma pour traiter des tissus vivants comprenant :
- une paire d'électrodes (6 et 9), l'une d'entre elles étant reliée à un potentiel de référence ;
- un générateur électrique (1) relié aux dites électrodes, adapté pour produire entre elles une différence de potentiel variable dans le temps ;
- un conduit, dont une extrémité est reliée à une source de gaz et l'autre extrémité contient lesdites électrodes, de façon que le plasma soit produit juste avant d'émerger du conduit, ***caractérisé en ce que*** ledit générateur électrique est un générateur électrique radiofréquence ayant une puissance comprise entre 1 et 30 W.

2. Un dispositif selon la revendication 1, où ladite source de gaz délivre un mélange gazeux.

3. Un dispositif selon la revendication 2, où ledit mélange gazeux contient des gaz rares, tels que l'Argon et/ou l'Hélium et/ou le Néon.

4. Un dispositif selon la revendication 2, où ledit mélange gazeux contient des gaz réactifs tels que l'Oxygène et/ou l'Azote et/ou de la vapeur et/ou d'autres gaz selon les applications spécifiques.

5. Un dispositif selon la revendication 1, où les deux électrodes sont une électrode interne et une électrode externe, qui sont électriquement isolées l'une de l'autre au moyen d'un tube isolant (7) et sont maintenues en position par une structure de support (8), ce en quoi les deux dites électrodes définissent deux buses concentriques (6, 9).

6. Un dispositif selon la revendication 1, où ledit générateur électrique (1) est un générateur haute fréquence.

7. Un dispositif selon la revendication 1, muni en outre d'un circuit de couplage (2) que sert à minimiser la puissance réfléchie vers le générateur électrique (1) et à augmenter la tension jusqu'à des valeurs adaptées pour ioniser le gaz.

8. Un dispositif selon la revendication 1, où ledit potentiel de référence est la masse.

9. Un dispositif selon la revendication 5, où ladite électrode interne (6) a une entrée qui est à l'intérieur de l'électrode externe (9).

10. Un dispositif selon la revendication 5, où l'entrée de l'électrode interne (6) est recouverte d'une grille réalisée en un matériau conducteur (10) connectée de façon galvanique à l'électrode interne.

11. Un dispositif selon la revendication 5, où ladite l'électrode externe (9) a une entrée qui est recouverte d'une grille réalisée en un matériau conducteur (11) connectée de façon galvanique à l'électrode externe.

12. Un dispositif selon l'une des revendications 10 et 11, où lesdites grilles (10, 11) sont plates ou concaves ou convexes.

13. Un dispositif selon la revendication 12, où la distance entre lesdites grilles est comprise entre quelques dixièmes de millimètre et quelques dizaines de millimètre.

14. Un dispositif selon la revendication 12, où ladite grille externe (11) est plate et ladite grille interne (10) est convexe, la convexité étant tournée vers la grille externe (11).

15. Un dispositif selon la revendication 5, où ledit tube isolant (7) est aussi long que la buse formant l'électrode externe (9).

16. Un dispositif selon la revendication 7, où ledit le générateur électrique a une puissance optimale de 10W.

17. Un dispositif selon la revendication 7, où l'ensemble comprenant le générateur électrique et le circuit de couplage est adapté pour produire une différence de potentiel entre lesdites électrodes de l'ordre du kilovolt et une fréquence comprise entre 1 et 30 MHz.

18. Un dispositif selon la revendication 1, où le débit gazeux traversant ledit conduit est compris entre 0,1 et 5 litres/minute.
